# EUROPÄISCHE PATENTANMELDUNG

(11) **EP 1 637 606 A2**
(43) Veröffentlichungstag der Anmeldung: **22.03.2006**
(21) Anmeldenummer: 05025035.6
(22) Anmeldetag: 30.05.2001
(51) Int. Cl.: C12N 15/82, C12N 15/55, C12N 9/20, C07K 14/415, C12Q 1/68, A01H 5/00

(54) **Triacylglycerol-Lipasen**

(30) Priorität: 31.05.2000 DE 10026845
(62) Teilanmeldung aus: 01957812.9
(71) Anmelder: IPK-Institut für Pflanzengenetik und Kulturpflanzen Forschung, 06466 Gatersleben (DE)
(72) Erfinder: Körner, Martina, 12687 Berlin (DE); Berndt, Ekkehart, 63179 Obertshausen (DE); Fritsche, Kathrin, 6811 HN Arnhem (NL); Feussner, Ivo, 37085 Göttingen (DE)
(74) Vertreter: Maiwald Patentanwalts GmbH

(57) **Zusammenfassung**

Die Erfindung betrifft Nukleinsäuremoleküle, die für Proteine mit der enzymatischen Aktivität einer Acylhydrolase, insbesondere einer Triacylglycerol-Lipase (TAG-Lipase) kodieren, Verfahren zur Erzeugung transgener Pflanzen, die diese Nukleinsäuremoleküle enthalten und deren Gehalt an Acylhydrolase, insbesondere an TAG-Lipase im Vergleich zu Wildtyppflanzen verändert ist, diese neuen Pflanzen, deren Teile und Produkte und Pflanzenzellen, sowie die Verwendung der Nukleinsäuremoleküle zur Beeinflussung des Gehalts an mehrfachungesättigten Fettsäuren in transgenen Pflanzen.

SEQUENZPROTOKOLL

<110> IPK - Institut für Pflanzengenetik und Kulturpflan

<120> Triacylglycerol-Lipasen

<130> I 7269

<140>
<141>

<150> DE 100 26 845
<151> 2000-05-31

<160> 14

<170> PatentIn Ver. 2.1

<210> 1
<211> 1331
<212> DNA
<213> Arabidopsis thaliana

<400> 1
ccatcacgga tccgcatgct cccggccgcc atggcggccg cgggaattcg attggggcat 60
gcatgtctga aaacagagag gcttggtcag cgaattccga ggaaatggag ctgcttcacg 120
gctccaacag attatcttca ccggaacatg ttcgtagaag agtctccggg aattcttctg 180
aggacggttc gcctcgaatt tgccggcagc agtcgttcgg tcgcgacata ggccatgcag 240
ctgccgagac gtatctcatt actcgactta gcttcaacct tcttggatat ctcggggtag 300
gttaccggtg gatcacaaga ttacttgctc ttgcttgtta tgcaatgctc cttatgcctg 360
gtttccttca agttgcatat ctttattttt tctcatctca agtcaggagg agtatagttt 420
atggaggtca tccaaggaat aggctggatt tgtacatacc gccaacgagt gatggcctga 480
agccggttgt agttttcgtt actggtggag cttggattat cggttacaaa gcttggggtt 540
ctctcttggg actacagcta gcagaaaggg acataattgt ggcatgtcta gattacagaa 600
actttcctca ggggacaatt agtgatatgg taagtgatgc agctcaagga atctcatttg 660
tctgcaataa catctctgca tttggaggtg atcctaacag aatctatctg atggggcaat 720
cggctggtgc acatatctct tcttgtgctc tctttgaaca agctattaaa gaatcaagag 780
gggagagcat ctcatggagt gtatctcaga ttaaagctta ctttggttta tctggagggt 840
acaatctctt taatctggtt gaacacttcc acaaccgagg cttgtatcgt tcaattttcc 900
taagcataat ggaaggagaa gagtccttta aacaattctc ccctgaagta agattgaaag 960
acctgaatgt aagaaaagct gcagccctct tacctcatat tatactcttt catggatccg 1020
cagattattc aatcccacct gaagcaagca aaacttttac agatgctctt caagctgctg 1080
aagtgaaagc tgagctagtt atgtacaagg gtaaaactca taccgaccta tttcttcagg 1140
atcctctgag aggcggtaaa gatgagctat ttgatcatat agtctctatg atacacgccg 1200
atgacagtga cgcattgaga aacgatgctg ttgcgccacc gaggaaacgg cttgttccag 1260
agttcttgct gaaactggct ggcagggtca gccctttttg agtcgacctg cagccaagct 1320
taattagctg a 1331

<210> 2
<211> 412
<212> PRT
<213> Arabidopsis thaliana

<400> 2
Met Ser Glu Asn Arg Glu Ala Trp Ser Ala Asn Ser Glu Glu Met Glu
1               5                   10                  15

Leu Leu His Gly Ser Asn Arg Leu Ser Ser Pro Glu His Val Arg Arg
            20                  25                  30

Arg Val Ser Gly Asn Ser Ser Glu Asp Gly Ser Pro Arg Ile Cys Arg
        35                  40                  45

Gln Gln Ser Phe Gly Arg Asp Ile Gly His Ala Ala Ala Glu Thr Tyr
    50                  55                  60

Leu Ile Thr Arg Leu Ser Phe Asn Leu Leu Gly Tyr Leu Gly Val Gly
65                  70                  75                  80

Tyr Arg Trp Ile Thr Arg Leu Leu Ala Leu Ala Cys Tyr Ala Met Leu
                85                  90                  95

Leu Met Pro Gly Phe Leu Gln Val Ala Tyr Leu Tyr Phe Phe Ser Ser
            100                 105                 110

Gln Val Arg Arg Ser Ile Val Tyr Gly Gly His Pro Arg Asn Arg Leu
        115                 120                 125

Asp Leu Tyr Ile Pro Pro Thr Ser Asp Gly Leu Lys Pro Val Val Val
    130                 135                 140

Phe Val Thr Gly Gly Ala Trp Ile Ile Gly Tyr Lys Ala Trp Gly Ser
145                 150                 155                 160

Leu Leu Gly Leu Gln Leu Ala Glu Arg Asp Ile Ile Val Ala Cys Leu
                165                 170                 175

Asp Tyr Arg Asn Phe Pro Gln Gly Thr Ile Ser Asp Met Val Ser Asp
            180                 185                 190

Ala Ala Gln Gly Ile Ser Phe Val Cys Asn Asn Ile Ser Ala Phe Gly
        195                 200                 205

Gly Asp Pro Asn Arg Ile Tyr Leu Met Gly Gln Ser Ala Gly Ala His
    210                 215                 220

Ile Ser Ser Cys Ala Leu Phe Glu Gln Ala Ile Lys Glu Ser Arg Gly
225                 230                 235                 240

Glu Ser Ile Ser Trp Ser Val Ser Gln Ile Lys Ala Tyr Phe Gly Leu
                245                 250                 255

Ser Gly Gly Tyr Asn Leu Phe Asn Leu Val Glu His Phe His Asn Arg
            260                 265                 270

Gly Leu Tyr Arg Ser Ile Phe Leu Ser Ile Met Glu Gly Glu Glu Ser
        275                 280                 285

Phe Lys Gln Phe Ser Pro Glu Val Arg Leu Lys Asp Leu Asn Val Arg
    290                 295                 300

Lys Ala Ala Ala Leu Leu Pro His Ile Ile Leu Phe His Gly Ser Ala
305                 310                 315                 320

Asp Tyr Ser Ile Pro Pro Glu Ala Ser Lys Thr Phe Thr Asp Ala Leu
                325                 330                 335

Gln Ala Ala Glu Val Lys Ala Glu Leu Val Met Tyr Lys Gly Lys Thr
            340                 345                 350

His Thr Asp Leu Phe Leu Gln Asp Pro Leu Arg Gly Gly Lys Asp Glu
        355                 360                 365

Leu Phe Asp His Ile Val Ser Met Ile His Ala Asp Asp Ser Asp Ala
    370                 375                 380

Leu Arg Asn Asp Ala Val Ala Pro Pro Arg Lys Arg Leu Val Pro Glu
385                 390                 395                 400

Phe Leu Leu Lys Leu Ala Gly Arg Val Ser Pro Phe
                405                 410

<210> 3
<211> 1155
<212> DNA
<213> Arabidopsis thaliana

<400> 3
atggcatctt cactgaagaa gcttatctca agctttctac ttgtcttata ctccaccacc 60
atcattgttg cttcatcaga atctcgatgt aggcgtttta aatcgatcat cagctttggt 120
gattccatcg cagacaccgg aaattatctc catctctctg atgtcaatca ccttcctcaa 180
tccgcctttc ttccttatgg cgaaagcttc ttccatcctc cctccggtcg tgcctctaat 240
ggccgtctca tcatcgactt cattgccgaa ttcttgggac taccatacgt accgccttac 300
tttggatccc aaaacgttag cttcgaacaa gggatcaatt ttgcggtgta tggagcaacc 360
gcattggacc gagcgtttct tttgggaaaa ggaattgaat ctgatttcac caatgttagt 420
ttaagtgttc agcttgacac cttcaagcag attttgccta acttatgcgc ctcgtctact 480
cgtgattgca aagagatgct tggagactcg ctgatactca tgggagagat tggaggaaac 540
gactataatt acccattttt tgaaggaaaa agtatcaatg aaatcaaaga gcttgttcct 600
ctaatcgtca aagctatttc ttctgctatt gtggatttga ttgatttagg gggcaaaaca 660
tttttggtac ccggaggctt cccaacagga tgttctgcgg cgtatcttac tctatttcag 720
accgtggcag aaaaagacca ggacccttta acaggttgtt acccattgct caacgaattt 780
ggcgagcacc acaacgaaca gctaaagaca gaactcaagc gactccaaaa attctatcct 840
catgtcaaca tcatttacgc tgactaccac aactccttat accggtttta tcaagaacca 900
gctaaatacg ggtttaagaa caaaccttta gctgcttgct gtggagtcgg aggtaaatac 960
aacttcacta ttggtaagga gtgtggatac gaaggagtta actattgtca aaatccttct 1020
gagtatgtga actgggatgg ttatcattta accgaagccg cctaccagaa gatgactgag 1080
ggtatactca acggtcccta tgcaactcct gctttcgact ggtcctgcct tggctctggt 1140
acagtggata cataa                                                  1155

<210> 4
<211> 384
<212> PRT
<213> Arabidopsis thaliana

<400> 4
Met Ala Ser Ser Leu Lys Lys Leu Ile Ser Ser Phe Leu Leu Val Leu
1               5                   10                  15

Tyr Ser Thr Thr Ile Ile Val Ala Ser Ser Glu Ser Arg Cys Arg Arg
            20                  25                  30

Phe Lys Ser Ile Ile Ser Phe Gly Asp Ser Ile Ala Asp Thr Gly Asn
        35                  40                  45

Tyr Leu His Leu Ser Asp Val Asn His Leu Pro Gln Ser Ala Phe Leu
    50                  55                  60

Pro Tyr Gly Glu Ser Phe Phe His Pro Pro Ser Gly Arg Ala Ser Asn
65                  70                  75                  80

Gly Arg Leu Ile Ile Asp Phe Ile Ala Glu Phe Leu Gly Leu Pro Tyr
                85                  90                  95

Val Pro Pro Tyr Phe Gly Ser Gln Asn Val Ser Phe Glu Gln Gly Ile
            100                 105                 110

Asn Phe Ala Val Tyr Gly Ala Thr Ala Leu Asp Arg Ala Phe Leu Leu
        115                 120                 125

Gly Lys Gly Ile Glu Ser Asp Phe Thr Asn Val Ser Leu Ser Val Gln
    130                 135                 140

Leu Asp Thr Phe Lys Gln Ile Leu Pro Asn Leu Cys Ala Ser Ser Thr
145                 150                 155                 160

Arg Asp Cys Lys Glu Met Leu Gly Asp Ser Leu Ile Leu Met Gly Glu
                165                 170                 175

Ile Gly Gly Asn Asp Tyr Asn Tyr Pro Phe Phe Glu Gly Lys Ser Ile
            180                 185                 190

Asn Glu Ile Lys Glu Leu Val Pro Leu Ile Val Lys Ala Ile Ser Ser
        195                 200                 205

Ala Ile Val Asp Leu Ile Asp Leu Gly Gly Lys Thr Phe Leu Val Pro
    210                 215                 220

Gly Gly Phe Pro Thr Gly Cys Ser Ala Ala Tyr Leu Thr Leu Phe Gln
225                 230                 235                 240

Thr Val Ala Glu Lys Asp Gln Asp Pro Leu Thr Gly Cys Tyr Pro Leu
                245                 250                 255

Leu Asn Glu Phe Gly Glu His His Asn Glu Gln Leu Lys Thr Glu Leu
            260                 265                 270

Lys Arg Leu Gln Lys Phe Tyr Pro His Val Asn Ile Ile Tyr Ala Asp
        275                 280                 285

Tyr His Asn Ser Leu Tyr Arg Phe Tyr Gln Glu Pro Ala Lys Tyr Gly
    290                 295                 300

Phe Lys Asn Lys Pro Leu Ala Ala Cys Cys Gly Val Gly Gly Lys Tyr
305                 310                 315                 320

Asn Phe Thr Ile Gly Lys Glu Cys Gly Tyr Glu Gly Val Asn Tyr Cys
                325                 330                 335

Gln Asn Pro Ser Glu Tyr Val Asn Trp Asp Gly Tyr His Leu Thr Glu
            340                 345                 350

Ala Ala Tyr Gln Lys Met Thr Glu Gly Ile Leu Asn Gly Pro Tyr Ala
        355                 360                 365

Thr Pro Ala Phe Asp Trp Ser Cys Leu Gly Ser Gly Thr Val Asp Thr
    370                 375                 380

<210> 5
<211> 1293
<212> DNA
<213> Arabidopsis thaliana

<400> 5
atgtgtagaa gatatttcgt gcatagttgt tttttacttc tgctgttact caactatttg 60
ccctttcgat ttaacttctc ttataatctt gataggtatc catatgaagc tattcgtgtt 120

gttacatctg atggatatgg tcttctcttg gaaaggatac caagacgaga tgcgaggaaa 180
gctgtttatt tgcagcatgg tgtaatggat tcttcaatgg gatgggtatc aaatggtgtt 240
gttggatcac cagcttttgc agcttatgat caaggctacg atgttttcct agggaacttt 300
cgtggtttag tttcaagaga tcatgtgaaa aaaaacatat cctcaaaaga tttctggcgt 360
tactccatca atgaacatgc aacggaagat atcccagcaa tgatagagaa gattcacgaa 420
atcaaaactt cagaactgaa actttatcag cctactatgg aagaggtagt aaacgaggat 480
caaccgtata agctttgtgt tgtatctcac agtttaggcg gtgccgcggt tctgatgtat 540
gtaatcaccc gtaaaatcga agagaaaccg cacagactct cgagactgat ccttctttcg 600
cctgctgggt ttcactatga ctccaacatg tgtttcacgt tgatggagta cacgttcctt 660
ttcttgggtc ctgtactatc tcggattgtt cctgctttct acatccccac taaattcttc 720
cggatgcttc tcaacaagtt agctcgagat ttccataact atcctgctgt tggtggatta 780
gtccaaacat tgatgagtta tgtagttggc ggagatagct caaactgggt tggagtcatg 840
ggattgcctc actataacat gaacgatatg ccaggtatat ccttccgtgt ggctcagcat 900
cttgcacaga ttaaacatag cggtaagttc aagatgtttg attacggtag cagttcagct 960
aatatggatg tttacgggtc ccctgagccg ctcgatcttg ggagttttta tgggttgatc 1020
gatgtgcctg tggatttagt agctggaaag aaagacaaag tgattagacc ttcaatggtt 1080
aggaaacatt acagggtgat gagagattca ggagtagatg tttcttacaa tgagtttgag 1140
tatgctcatt tggattttac cttctctcac cgtgaagagc ttttggcgta tgtgatgtcg 1200
cggttacttc tcgtggagcc aacgcagact cagacggttc ataagaaggg gatgaagctg 1260
aagaagaaaa tggaaacagg caaacctcat ctg 1293

<210> 6
<211> 431
<212> PRT
<213> Arabidopsis thaliana

<400> 6
Met Cys Arg Arg Tyr Phe Val His Ser Cys Phe Leu Leu Leu Leu Leu
1               5                   10                  15

Leu Asn Tyr Leu Pro Phe Arg Phe Asn Phe Ser Tyr Asn Leu Asp Arg
            20                  25                  30

Tyr Pro Tyr Glu Ala Ile Arg Val Val Thr Ser Asp Gly Tyr Gly Leu
        35                  40                  45

Leu Leu Glu Arg Ile Pro Arg Arg Asp Ala Arg Lys Ala Val Tyr Leu
    50                  55                  60

Gln His Gly Val Met Asp Ser Ser Met Gly Trp Val Ser Asn Gly Val
65                  70                  75                  80

Val Gly Ser Pro Ala Phe Ala Ala Tyr Asp Gln Gly Tyr Asp Val Phe
                85                  90                  95

Leu Gly Asn Phe Arg Gly Leu Val Ser Arg Asp His Val Lys Lys Asn
            100                 105                 110

Ile Ser Ser Lys Asp Phe Trp Arg Tyr Ser Ile Asn Glu His Ala Thr
        115                 120                 125

Glu Asp Ile Pro Ala Met Ile Glu Lys Ile His Glu Ile Lys Thr Ser
    130                 135                 140

Glu Leu Lys Leu Tyr Gln Pro Thr Met Glu Glu Val Val Asn Glu Asp
145                 150                 155                 160

Gln Pro Tyr Lys Leu Cys Val Val Ser His Ser Leu Gly Gly Ala Ala
                165                 170                 175

Val Leu Met Tyr Val Ile Thr Arg Lys Ile Glu Glu Lys Pro His Arg
            180                 185                 190

Leu Ser Arg Leu Ile Leu Leu Ser Pro Ala Gly Phe His Tyr Asp Ser
        195                 200                 205

Asn Met Cys Phe Thr Leu Met Glu Tyr Thr Phe Leu Phe Leu Gly Pro
    210                 215                 220

Val Leu Ser Arg Ile Val Pro Ala Phe Tyr Ile Pro Thr Lys Phe Phe
225                 230                 235                 240

Arg Met Leu Leu Asn Lys Leu Ala Arg Asp Phe His Asn Tyr Pro Ala
                245                 250                 255

Val Gly Gly Leu Val Gln Thr Leu Met Ser Tyr Val Val Gly Gly Asp
            260                 265                 270

Ser Ser Asn Trp Val Gly Val Met Gly Leu Pro His Tyr Asn Met Asn
        275                 280                 285

Asp Met Pro Gly Ile Ser Phe Arg Val Ala Gln His Leu Ala Gln Ile
    290                 295                 300

Lys His Ser Gly Lys Phe Lys Met Phe Asp Tyr Gly Ser Ser Ser Ala
305                 310                 315                 320

Asn Met Asp Val Tyr Gly Ser Pro Glu Pro Leu Asp Leu Gly Glu Phe
                325                 330                 335

Tyr Gly Leu Ile Asp Val Pro Val Asp Leu Val Ala Gly Lys Lys Asp
            340                 345                 350

Lys Val Ile Arg Pro Ser Met Val Arg Lys His Tyr Arg Val Met Arg
        355                 360                 365

Asp Ser Gly Val Asp Val Ser Tyr Asn Glu Phe Glu Tyr Ala His Leu
    370                 375                 380

Asp Phe Thr Phe Ser His Arg Glu Glu Leu Leu Ala Tyr Val Met Ser
385                 390                 395                 400

Arg Leu Leu Leu Val Glu Pro Thr Gln Thr Gln Thr Val His Lys Lys
                405                 410                 415

Gly Met Lys Leu Lys Lys Lys Met Glu Thr Gly Lys Pro His Leu
            420                 425                 430


<210> 7
<211> 33
<212> DNA
<213> Künstliche Sequenz

<220>
<223> Beschreibung der künstlichen Sequenz:
      Oligonukleotidprimer

<400> 7
cccgcatgca tgcagttgtc tccggaacga tgc                                33


<210> 8
<211> 33
<212> DNA

<213> Künstliche Sequenz

<220>
<223> Beschreibung der künstlichen Sequenz:
      Oligonukleotidprimer

<400> 8
ggggcatgca tgtctgaaaa cagagaggct tgg                                    33


<210> 9
<211> 33
<212> DNA
<213> Künstliche Sequenz

<220>
<223> Beschreibung der künstlichen Sequenz:
      Oligonukleotidprimer

<400> 9
aaagcatgca tggagctgct tcacggctcc aac                                    33


<210> 10
<211> 34
<212> DNA
<213> Künstliche Sequenz

<220>
<223> Beschreibung der künstlichen Sequenz:
      Oligonukleotidprimer

<400> 10
aaagtcgact caaaaagggc tgaccctgcc agcc                                   34


<210> 11
<211> 30
<212> DNA
<213> Künstliche Sequenz

<220>
<223> Beschreibung der künstlichen Sequenz:
      Oligonukleotidprimer

<400> 11
ccccgcatgc atggcttctt cactgaagaa                                        30


<210> 12
<211> 36
<212> DNA
<213> Künstliche Sequenz

<220>
<223> Beschreibung der künstlichen Sequenz:
      Oligonukleotidprimer

<400> 12
aaactgcagt tatgtatcca ctgtaccaga gccaag                                 36


<210> 13

<211> 36
<212> DNA
<213> Künstliche Sequenz

<220>
<223> Beschreibung der künstlichen Sequenz:
      Oligonukleotidprimer

<400> 13
ggggcatgca tgtgtagaag atatttcgtg catagt                                36


<210> 14
<211> 34
<212> DNA
<213> Künstliche Sequenz

<220>
<223> Beschreibung der künstlichen Sequenz:
      Oligonukleotidprimer

<400> 14
tttctgcagt gacagatgag gtttgcctgt ttcc                                  34

## Beschreibung

Die Erfindung betrifft Nukleinsäuremoleküle, die für Proteine mit der enzymatischen Aktivität einer Acylhydrolase, insbesondere einer Triacylglycerol-Lipase (TAG-Lipase) kodieren, Verfahren zur Erzeugung transgener Pflanzen, die diese Nukleinsäuremoleküle enthalten und deren Gehalt an Acylhydrolase, insbesondere TAG-Lipase im Vergleich zu Wildtyppflanzen verändert ist, diese neuen Pflanzen, deren Teile und Produkte und Pflanzenzellen, sowie die Verwendung der Nukleinsäuremoleküle zur Beeinflussung des Gehalts an mehrfach ungesättigten Fettsäuren in transgenen Pflanzen.

Lipasen katalysieren eine große Anzahl an Reaktionen, von denen viele ein industrielles Potential aufweisen. TAG-Lipasen katalysieren die Umwandlung von Triacylglycerol und Wasser in Diacylglycerol und ein Fettsäureanion.

Die Aminosäuresequenzen von Lipasen aus dem menschlichen Magen, aus der Rattenzunge und aus dem menschlichen hepatischen Lysosom sind homolog, aber bis auf eine Sequenz von sechs Aminosäuren um die wesentliche Aminosäure Serin 152 der Lipase aus dem Schweinepankreas (M. W. Bodner (1987) *Biochem. Biophys. Acta* 909: 237-244) nicht mit der Lipase aus dem Schweinepankreas verwandt. Diese Enzyme sind glycosyliert, enthalten ein hydrophobes Signalpeptid und gehören der Familie der sauren Lipasen an (D. Ameis et al. (1994) *Eur. J. Biochem.* 219: 905-914). Die lysosomale saure Lipase (lysosomal acid lipase, LAL) ist eine für den intrazellulären Abbau von Cholesterylestern und Triacylglycerolen wesentliche Hydrolase und spielt eine Rolle bei der Mobilisierung des Saatöls während der Keimung.

Neutrale Triacylglycerol-Lipasen wurden in Pilzen, Bakterien, Säugetieren und Insekten in großem Ausmaß untersucht. Es wurden Nukleotidsequenzen mit Ähnlichkeiten zu den neutralen Triacylglycerol-Lipasen in *Arabidopsis thaliana* und *Ipomea nil* beschrieben, aber ihre Funktion wurde nicht bewiesen. Ferner wurde die Kristallstruktur von *Mucor miehei* Triacylglycerol-Lipase berichtet, bei der eine Trypsin-artige katalytische Triade Ser-His-Asp mit einem aktiven Serinrest unterhalb eines kurzen helikalen Fragments eines langen Oberflächen-Loops gefunden wurde (L. Brady et al. (1990) *Nature* 343: 767-770).

Es wird angenommen, daß die hauptsächliche physiologische Funktion der pflanzlichen TAG-Lipasen die Mobilisierung von Speicherlipiden während des Keimungsprozesses ist.

Besonders für Ölsaaten stellen die Speicherlipide die Hauptkohlenstoffquelle des wachsenden Keimlings dar. Im Verlauf der Keimung lassen sich bestimmte pflanzliche Lipoxygenasen (LOXs) an der Membran der Lipidkörper verschiedener Ölsaaten nachweisen. Sie überführen spezifisch Polyenfettsäurereste, d. h. mehrfach ungesättigte Fettsäurereste, von Triacylglycerinen direkt in Hydroperoxy- oder Ketodienfettsäurederivate. Die aus dieser Reaktion resultierenden Metabolite, d. h. Triacylglycerine, deren Acylreste aus 13-Hydro(pero)xylinolsäure bestehen, werden anschließend von einer TAG-Lipase hydrolysiert, die hochspezifisch für oxidierte Polyenfettsäurereste ist. Diese derart modifizierten Fettsäuren dienen dem wachsenden Keimling als Energiereserve.

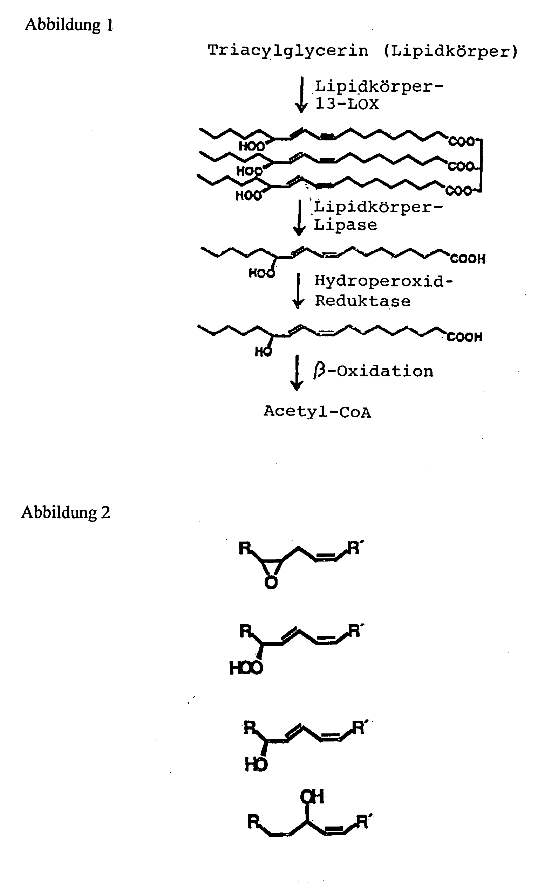

Wie bereits vorstehend erwähnt, wird das Auftreten von LOXs von der Akkumulation von Hydro(pero)xy-Derivaten begleitet, welche im Gegensatz zu den nicht-oxygenierten mehrfach ungesättigten Fettsäureresten (PUFA-Reste) von den Speicherlipiden abgespalten werden. Aus diesen Beobachtungen wird geschlossen, daß diese durch die LOXs katalysierte Oxidationsreaktion den Katabolismus von mehrfach ungesättigten Fettsäuren (Reduktase-Weg) in Pflanzen initiiert (siehe Abb. 1). Ferner wurde die spezifische TAG-Lipase aus keimenden Gurkenkeimlingen gereinigt, und ihre biochemischen Eigenschaften wurden bestimmt. Diese Lipidkörper-assoziierte TAG-Lipase zeigte tatsächlich eine hohe Spezifität für oxidierte mehrfach ungesättigte Fettsäuren mit den in Abbildung 2 gezeigten Strukturmotiven. Weiter wurde diese spezielle TAG-Lipase in der Lipidkörperfraktion von sich entwickelnden Keimlingen gefunden, was eine zumindest zweifache physiologische Funktion dieser Enzyme in Pflanzen nahelegt.

Die Isolierung von pflanzlichen TAG-Lipasen kann für die Verarbeitung von Pflanzenölen nützlich sein, die Fettsäuren mit unüblichen oxygenierten Strukturen enthalten. Derartige pflanzliche Öle sind für die Verwendung in der Lebensmittelindustrie problematisch, da die in ihnen enthaltenen oxygenierten Fettsäuren zu einer geringeren Haltbarkeit führen. Mit Hilfe von spezifischen TAG-Lipasen können diese Fettsäurereste aus den entsprechenden Ölen selektiv entfernt werden. Die freigesetzten oxygenierten Fettsäuren stellen wiederum wichtige Copolymere bei der Herstellung von Kunststoffen dar.

Damit können die definiert strukturierten Lipide mit oxygenierten Fettsäureresten an bestimmten Positionen des Glycerol-Rückgrats nach der Erzeugung der mehrfach ungesättigten Fettsäuren, die in Triacylglycerolen verestert sind, durch Entfernen der kontaminierenden Lipidperoxide gereinigt werden. Umgekehrt können bestimmte oxygenierte Fettsäuren in bestimmte Positionen von TAGs eingeführt werden.

Transgene Pflanzen, die große Mengen an diesen oxygenierten Fettsäuren innerhalb ihres Samenöls akkumulieren, benötigen diese spezifische Lipase für die Keimung. Darüber hinaus kann durch die gemeinsame Expression einer TAG-LOX und einer TAG-Lipase im Samen der Gehalt an PUFAs im Samenöl verringert werden.

Demzufolge ist es eine wesentliche Aufgabe der vorliegenden Erfindung, ein Verfahren bereitzustellen, mit dem der Gehalt an mehrfach ungesättigten Fettsäuren, insbesondere an oxygenierten, mehrfach ungesättigten Fettsäuren in Pflanzen vermindert oder erhöht werden kann. Ferner ist es eine Aufgabe der vorliegenden Erfindung, Nukleinsäuremoleküle bereitzustellen, die auf Pflanzenzellen oder Pflanzensamen übertragen werden können, um den Gehalt an Polyenfettsäuren, insbesondere an oxidierten Polyenfettsäureresten zu beeinflussen. Weitere Aufgaben der Erfindung ergeben sich aus der folgenden Beschreibung.

Diese Aufgaben werden durch die Gegenstände der unabhängigen Ansprüche, insbesondere basierend auf der Bereitstellung der erfindungsgemäßen DNA-Sequenzen, deren Genprodukte unmittelbar an der Hydrolyse von Triacylglycerinen mit oxygenierten, ungesättigten Fettsäureresten beteiligt sind, und der in einem modifizierten Gehalt an derartigen Fettsäuren resultierenden Übertragung der DNA-Sequenzen auf Pflanzen, gelöst.

Vorteilhafte Ausgestaltungen sind in den Unteransprüchen definiert.

Die vorliegende Erfindung umfaßt somit rekombinante Nukleinsäuremoleküle, die
a) regulatorische Sequenzen eines in Pflanzenzellen aktiven Promotors;
b) operativ daran gebunden eine DNA-Sequenz, die ein Protein mit der enzymatischen Aktivität einer Acylhydrolase, insbesondere einer Triacylglycerol-Lipase (TAG-Lipase), besonders bevorzugt einer für oxygenierte Polyenfettsäuren spezifischen TAG-Lipase kodiert; und
c) operativ daran gebunden regulatorische Sequenzen, die als Transkriptions-, Terminations- und/oder Polyadenylierungssignale in Pflanzenzellen dienen können,
umfassen.

Sequenzen, die eine TAG-Lipase kodieren, sind im Stand der Technik beschrieben worden, ohne daß allerdings deren Funktion nachgewiesen wurde. So offenbart PCT/US99/09280 unter anderem die Sequenzen von TAG-Lipase-cDNA-Klons aus Mais, Reis und Soya.

Es ist bei der vorliegenden Erfindung überraschenderweise erstmals gelungen, Nukleinsäuremoleküle bereitzustellen, die ein Protein mit der enzymatischen Aktivität einer TAG-Lipase kodieren. Ferner wird erfindungsgemäß erstmals die Überexpression eines durch die vorstehend genannten Nukleinsäuremoleküle kodierten Proteins mit TAG-Lipase-Aktivität in Pflanzen beschrieben. Die erfindungsgemäßen TAG-Lipasen zeigen eine hohe Spezifität für oxygenierte Polyenfettsäuren, hydrolysieren aber unter bestimmten Reaktionsbedingungen auch normale TAGs.

Insbesondere betrifft die Erfindung pflanzliche DNA-Sequenzen, die für ein Protein mit der biologischen Aktivität einer Acylhydrolase, insbesondere einer TAG-Lipase oder ein biologisch aktives Fragment davon aus *Arabidopsis* kodieren. Besonders bevorzugt betrifft die Erfindung die im beigefügten Sequenzprotokoll unter SEQ ID No: 1, SEQ ID No:3 und SEQ ID No:5 angegebenen Sequenzen, die abgeleiteten Aminosäuresequenzen sind unter SEQ ID No:2 , SEQ ID No:4 bzw. SEQ ID No: 6 angegeben.

Biologisch aktives Fragment bedeutet im Rahmen dieser Erfindung, daß die vermittelte biologische Aktivität zu einer Beeinflussung des Gehalts an mehrfach ungesättigten Fettsäuren, insbesondere an mehrfach ungesättigten oxygenierten Fettsäuren ausreicht.

Die DNA-Sequenz, die ein Protein mit der biologischen Aktivität einer Acylhydrolase, insbesondere einer TAG-Lipase, besonders bevorzugt einer für oxygenierte Polyenfettsäuren spezifischen TAG-Lipase kodiert, kann aus natürlichen Quellen isoliert oder nach herkömmlichen Verfahren synthetisiert werden. Mittels gängiger molekularbiologischer Techniken (siehe beispielsweise Sambrook *et al.* (1989) Molecular Cloning: A Laboratory Manual, 2. Auflage, Cold Spring Harbor Laboratory Press, Cold Spring Harbor, New York) ist es möglich, gewünschte Konstrukte für die Transformation von Pflanzenzellen vorzubereiten bzw. herzustellen. Die für die gentechnische Manipulation in prokaryontischen Zellen üblicherweise eingesetzten Klonierungs-, Mutagenisierungs-, Sequenzanalyse-, Restriktionsanalyse- und weitere biochemisch-molekularbiologischen Methoden sind dem Durchschnittsfachmann wohl bekannt. So können nicht nur geeignete chimäre Genkonstrukte mit der gewünschten Fusion von Promotor und erfindungsgemäßer TAG-Lipase-DNA-Sequenz und ggf. weiteren Regulations- und/oder Signalsequenzen hergestellt werden, vielmehr kann der Fachmann, falls erwünscht, zusätzlich mittels Routinetechniken, verschiedenartige Mutationen in die die erfindungsgemäße TAG-Lipase kodierende DNA-Sequenz einführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletion vom 5'- oder vom 3'-Ende der kodierenden DNA-Sequenz die Synthese entsprechend verkürzter Proteine erreicht werden kann. Ferner ist es möglich, gezielt Enzyme herzustellen, die durch Addition entsprechender Signalsequenzen in bestimmten Kompartimenten der Pflanzenzelle lokalisiert sind. Derartige Sequenzen sind in der Literatur beschrieben und dem Durchschnittsfachmann wohl bekannt (siehe z.B. Braun *et al.* (1992) EMBO J. 11:3219-3227; Wolter F. *et al.* (1988) Proc. Natl. Acad. Sci. USA 85:846-850; Sonnewald U. *et al.* (1991) Plant J. 1:95-106). Weiterhin ist auch die Einführung von Punktmutationen an Positionen denkbar, bei denen eine Veränderung der Aminosäuresequenz einen Einfluss beispielsweise auf die Enzymaktivität oder die Regulierung des Enzyms hat. Auf diese Weise können z.B. Mutanten hergestellt werden, die nicht mehr den normalerweise in der Zelle herrschenden Regulationsmechanismen über allosterische Regulation oder kovalente Modifizierung unterliegen. Des weiteren können Mutanten hergestellt werden, die eine veränderte Substrat- oder Produktspezifität aufweisen. Weiterhin können Mutanten hergestellt werden, die ein verändertes Aktivitäts-, Temperatur- und/oder pH-Profil aufweisen.

Für die gentechnische Manipulation in prokaryontischen Zellen können die erfindungsgemäßen rekombinanten Nukleinsäuremoleküle oder Teile davon in Plasmide eingebracht werden, die eine Mutagenese oder eine Sequenzveränderung durch Rekombination von DNA-Sequenzen erlauben. Mit Hilfe von Standardverfahren (siehe z.B. Sambrook *et al.* (1989), vide supra) können Basenaustausche vorgenommen oder natürliche oder synthetische Sequenzen hinzugefügt werden. Für die Verbindung der DNA-Fragmente untereinander können an die Fragmente, wo erforderlich, Adapter oder Linker angefügt werden. Ferner können mittels enzymatischer und anderer Manipulationen passende Restriktionsschnittstellen zur Verfügung gestellt oder überflüssige DNA oder Restriktionsschnittstellen entfernt werden. Wo Insertionen, Deletionen oder Substitutionen in Frage kommen, können in vitro-Mutagenese, "primer repair", Restriktion oder Ligation verwendet werden. Als Analysemethoden werden im allgemeinen Sequenzanalyse, Restriktionsanalyse und weitere biochemisch-molekularbiologische Methoden durchgeführt.

In einer bevorzugten Ausführungsform ist die DNA-Sequenz, die ein Protein mit der biologischen Aktivität einer Acylhydrolase, insbesondere einer TAG-Lipase, besonders bevorzugt einer für oxygenierte Polyenfettsäuren spezifischen TAG-Lipase, kodiert, ausgewählt aus der Gruppe, bestehend aus
a) DNA-Sequenzen, die die in SEQ ID No. 1 angegebene Nukleotidsequenz umfassen,
b) DNA-Sequenzen, die die in SEQ ID No. 3 angegebene Nukleotidsequenz umfassen,
c) DNA-Sequenzen, die die in SEQ ID No. 5 angegebene Nukleotidsequenz umfassen,
d) DNA-Sequenzen, die eine Nukleotidsequenz umfassen, die die in SEQ ID No. 2 angegebene Aminosäuresequenz oder Fragmente davon kodieren,
e) DNA-Sequenzen, die die in SEQ ID No. 4 angegebene Aminosäuresequenz oder Fragmente davon kodieren,
f) DNA-Sequenzen, die die in SEQ ID No. 6 angegebene Aminosäuresequenz oder Fragmente davon kodieren,
g) DNA-Sequenzen, die eine Nukleotidsequenz, die mit einem komplementären Strang der Nukleotidsequenz von a), b), c), d), e) oder f) hybridisieren, oder Fragmente dieser Nukleotidsequenz umfassen,
h) DNA-Sequenzen, die eine Nukleotidsequenz, die zu einer Nukleotidsequenz von g) degeneriert ist, oder Fragmente dieser Nukleotidsequenz umfassen,
i) DNA-Sequenzen, die ein Derivat, Analog oder Fragment einer Nukleotidsequenz von a), b), c), d), e), f), g) oder h) darstellen.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook *et al.* (1989, vide supra) beschrieben sind.

DNA-Sequenzen, die mit den DNA-Sequenzen hybridisieren, welche ein pflanzliches Protein mit der biologischen Aktivität einer TAG-Lipase, insbesondere einer für oxygenierte Polyenfettsäuren spezifischen TAG-Lipase, kodieren, können z.B. aus genomischen oder cDNA-Bibliotheken einer beliebigen Pflanze, die die erfindungsgemäßen TAG-Lipase-DNA-Sequenzen natürlicherweise besitzt, isoliert werden. Die Identifizierung und Isolierung derartiger DNA-Sequenzen kann dabei z.B. unter Verwendung von DNA-Sequenzen erfolgen, die exakt oder im wesentlichen die in SEQ ID No. 1, SEQ ID No. 3 oder SEQ ID No. 5 angegebene Nukleotidsequenz oder Teile davon aufweisen, bzw. der reversen Komplemente dieser DNA-Sequenzen, z.B. mittels Hybridisierung nach Standardverfahren (siehe z.B. Sambrook *et al.* (1989), vide supra). Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit einer der oben erwähnten TAG-Lipase-DNA-Sequenzen oder einem Teil davon übereinstimmt.

Die DNA-Sequenzen, die ein Protein mit der biologischen Aktivität einer Acylhydrolase, insbesondere einer TAG-Lipase, besonders bevorzugt einer für oxygenierte Polyenfettsäuren spezifischen TAG-Lipase kodieren, umfassen auch DNA-Sequenzen, deren Nukleotidsequenzen zu der einer der vorstehend beschriebenen DNA-Sequenzen degeneriert ist. Die Degeneration des genetischen Codes bietet dem Fachmann u.a. die Möglichkeit, die Nukleotidsequenz der DNA-Sequenz an die Codonpräferenz (codon usage) der Zielpflanze, also der aufgrund der Expression der erfindungsgemäßen TAG-Lipase einen veränderten Gehalt an mehrfach ungesättigten Fettsäuren, insbesondere an oxygenierten Polyenfettsäuren aufweisenden Pflanze bzw. Pflanzenzelle anzupassen und die Expression dadurch zu optimieren.

Die oben beschriebenen DNA-Sequenzen umfassen auch Fragmente, Derivate und allelische Varianten der oben beschriebenen DNA-Sequenzen, die ein Protein mit der biologischen Aktivität einer Acylhydrolase, insbesondere einer TAG-Lipase, besonders bevorzugt einer für oxygenierte Polyenfettsäuren spezifischen TAG-Lipase, kodieren. Unter "Fragmenten" werden dabei Teile der DNA-Sequenz verstanden, die lang genug sind, um eines der beschriebenen Proteine zu kodieren. Der Ausdruck "Derivat" bedeutet in diesem Zusammenhang, dass die Sequenzen sich von den oben beschriebenen DNA-Sequenzen an einer oder mehreren Positionen unterscheiden, aber einen hohen Grad an Homologie zu diesen Sequenzen aufweisen. Homologie bedeutet dabei eine Sequenzidentität von mindestens 25 Prozent, insbesondere eine Identität von mindestens 40 Prozent, vorzugsweise von mindestens 60, besonders bevorzugt von über 80 Prozent und am meisten bevorzugt von über 90 Prozent. Dabei weisen die durch diese DNA-Sequenzen kodierten Proteine eine Sequenzidentität zu den in SEQ ID No. 2, 4 respektive 6 angegebenen Aminosäuresequenzen von mindestens 60 Prozent, insbesondere mindestens 80 Prozent, vorzugsweise 85 Prozent und besonders bevorzugt über 90 Prozent, 95 Prozent und 98 Prozent auf. Die Abweichungen zu den oben beschriebenen DNA-Sequenzen können dabei beispielsweise durch Deletion, Substitution, Insertion oder Rekombination entstanden sein.

Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

In einer besonders bevorzugten Ausführungsform stammt die beschriebene, für eine Acylhydrolase, insbesondere für eine TAG-Lipase, besonders bevorzugt für eine für oxygenierte Polyenfettsäuren spezifische TAG-Lipase kodierende DNA-Sequenz aus *Arabidopsis thaliana.*

Weiter betrifft die Erfindung Nukleinsäuremoleküle, die die erfindungsgemäßen Nukleinsäuresequenzen enthalten oder durch natürlich vorkommende oder durch gentechnische oder chemische Prozesse und Syntheseverfahren aus diesen entstanden sind bzw. von diesen abgeleitet wurden. Hierbei kann es sich beispielsweise um DNA- oder RNA-Moleküle, cDNA, genomische DNA, mRNA usw. handeln.

Die Erfindung betrifft ebenfalls solche Nukleinsäuremoleküle, in denen die erfindungsgemäßen Nukleinsäuresequenzen mit regulatorischen Elementen verknüpft sind, die die Transkription und, falls erwünscht, die Translation in der Pflanzenzelle gewährleisten.

Für die Expression der in den erfindungsgemäßen rekombinanten Nukleinsäuremolekülen enthaltenen DNA-Sequenzen in pflanzlichen Zellen kommt grundsätzlich jeder in pflanzenlichen Zellen aktive Promotor in Frage. So können die erfindungsgemäßen DNA-Sequenzen beispielsweise unter Kontrolle konstitutiver, aber auch induzierbarer oder gewebe- bzw. entwicklungsspezifischer Regulationselemente, insbesondere Promotoren, in Pflanzenzellen exprimiert werden. Während beispielsweise die Verwendung eines induzierbaren Promotors die gezielt ausgelöste Expression der erfindungsgemäßen DNA-Sequenzen in Pflanzenzellen ermöglicht, bietet beispielsweise der Einsatz von gewebespezifischen, beispielsweise blatt- oder samenspezifischen, Promotoren die Möglichkeit, den Gehalt an oxygenierten Polyenfettsäuren in bestimmtem Gewebe, z.B. in Blatt- bzw. Samengewebe, zu verändern. Andere geeignete Promotoren vermitteln z.B. Lichtinduzierte Genexpression in transgenen Pflanzen. In Bezug auf die zu transformierende Pflanzen kann der Promotor homolog oder heterolog sein.

Geeignete Promotoren sind z.B. der 35S RNA-Promotor des Cauliflower Mosaic Virus und der Ubiquitin-Promotor aus Mais für eine konstitutive Expression. Als samenspezifische Promotoren bieten sich bspw. der USP- (Bäumlein et al. (1991), Mol. Gen. Genet. 225: 459-467) oder dem Hordein-Promotor (Brandt et al. (1985), Carlsberg Res. Commun. 50: 333-345).

Konstitutive keimungsspezifische und samenspezifische Promotoren werden im Rahmen dieser Erfindung bevorzugt, da sie sich besonders für die gezielte Erhöhung des Gehalts an oxygenierten Polyenfettsäuren in transgenen Samen eignen, u.a. auch im Zusammenhang mit der Antisense- oder Cosuppressions-Technik.

In jedem Fall kann der Fachmann geeignete Promotoren der Literatur entnehmen oder mittels Routineverfahren selbst aus beliebigen Pflanzen isolieren.

Ferner sind Transkriptions- bzw. Terminationssequenz vorhanden, die der korrekten Beendigung der Transkription dient, sowie der Addition eines polyA-Schwanzes an das Transkript dienen kann, dem eine Funktion bei der Stabilisierung der Transkripte beigemessen wird. Derartige Elemente sind in der Literatur beschrieben (z.B. Gielen (1989) EMBO J. 8:23-29) und beliebig austauschbar, z.B. der Terminator des Octopinsynthasegens aus *Agrobacterium tumefaciens.*

Die Erfindung betrifft weiterhin Proteine mit der biologischen Aktivität einer Acylhydrolase, insbesondere einer TAG-Lipase, besonders bevorzugt einer für oxygenierte Polyenfettsäuren spezifischen TAG-Lipase oder biologisch aktive Fragmente davon, die durch eine erfindungsgemäße Nukleinsäuresequenz oder ein erfindungsgemäßes Nukleinsäuremolekül kodiert werden. Vorzugsweise handelt es sich um eine pflanzliche TAG-Lipase aus *Arabidopsis thaliana*, besonders bevorzugt um ein Protein mit der in SEQ ID NO:2, SEQ ID NO:4 oder SEQ ID NO:6 gezeigten Aminosäuresequenz oder einem aktiven Fragment davon.

Eine weitere Aufgabe der Erfindung besteht darin, Vektoren bereitzustellen, deren Verwendung die Herstellung neuer Pflanzen, in denen ein veränderter Gehalt an mehrfach ungesättigten oxygenierten Fettsäuren erzielt werden kann, ermöglicht. Diese Aufgabe wird durch die Bereitstellung der erfindungsgemäßen Vektoren gelöst, die für Enzyme mit der Aktivität einer Acylhydrolase, insbesondere einer TAG-Lipase, besonders bevorzugt einer für oxygenierte Polyenfettsäuren spezifischen TAG-Lipase kodierende Nukleinsäuresequenzen enthalten.

Die vorliegende Erfindung betrifft somit auch Vektoren, insbesondere Plasmide, Kosmide, Viren, Bakteriophagen und andere in der Gentechnik gängige Vektoren, die die vorstehend beschriebenen erfindungsgemäßen Nukleinsäuremoleküle enthalten und ggf. für den Transfer der erfindungsgemäßen Nukleinsäuremoleküle auf Pflanzen bzw. Pflanzenzellen eingesetzt werden können.

In einer bevorzugten Ausführungsform sind die in den Vektoren enthaltenen Nukleinsäuremoleküle mit regulatorischen Elementen verknüpft, die die Transkription und ggf. Translation in prokaryontischen und eukaryontischen Zellen gewährleisten.

Gegegebenfalls können die Nukleinsäuresequenzen der Erfindung durch Enhancer-Sequenzen oder andere regulatorische Sequenzen ergänzt sein. Diese regulatorischen Sequenzen beinhalten beispielsweise auch Signalsequenzen, die für den Transport des Genprodukts zu einem bestimmten Kompartiment sorgen.

Es ist ebenso eine Aufgabe der Erfindung, neue transgene Pflanzen, Pflanzenzellen, Pflanzenteile, transgenes Vermehrungsmaterial und transgene Ernteprodukte bereitzustellen, die sich durch eine gegenüber Wildtyppflanzen bzw. -zellen veränderten Gehalt an Polyenfettsäuren, insbesondere an oxygenierten Polyenfettsäuren im Saatöl auszeichnen.

Diese Aufgabe wird durch die Übertragung der erfindungsgemäßen Nukleinsäuremoleküle und ihre Expression in Pflanzen gelöst. Durch die Bereitstellung der erfindungsgemäßen Nukleinsäuremoleküle besteht nun die Möglichkeit, pflanzliche Zellen mittels gentechnischer Methoden dahingehend zu verändern, daß sie im Vergleich zu Wildtypzellen eine neue oder veränderte TAG-Lipase-Aktivität aufweisen und es als Folge davon zu einer Veränderung des Gehalts an Polyenfettsäuren, insbesondere an oxygenierten Polyenfettsäuren im Saatöl kommt.

So betrifft die Erfindung in einer Ausführungsform Pflanzen bzw. deren Zellen und Teile, in denen der Gehalt an Polyenfettsäuren, insbesondere an oxygenierten Polyenfettsäuren im Saatöl aufgrund der Gegenwart und Expression der erfindungsgemäßen Nukleinsäuremoleküle gegenüber Wildtyppflanzen verringert ist.

Die Erfindung betrifft aber auch solche Pflanzen, in denen die Übertragung der erfindungsgemäßen Nukleinsäuremoleküle zu einer Erhöhung des Gehalts an Polyenfettsäuren, insbesondere an oxygenierten Polyenfettsäuren im Saatöl führt. Eine derartige Reduktion kann beispielsweise durch den Transfer von Antisense-Konstrukten oder durch andere Suppressionsmechanismen, wie beispielsweise Cosuppressionen, erreicht werden.

Gegenstand der Erfindung sind weiterhin transgene Pflanzenzellen bzw. solche Pflanzenzellen umfassende Pflanzen und deren Teile und Produkte, in denen die erfindungsgemäßen Nukleinsäuremoleküle integriert in das pflanzliche Genom vorliegen. Ebenfalls Gegenstand der Erfindung sind Pflanzen, in deren Zellen die erfindungsgemäße Nukleinsäuresequenz in selbstreplizierender Form vorliegt, d.h. die Pflanzenzelle enthält die fremde DNA auf einem eigenständigen Nukleinsäuremolekül (transiente Expression).

Bei den Pflanzen, die mit den erfindungsgemäßen Nukleinsäuremolekülen transformiert sind und in denen aufgrund der Einführung eines solchen Moleküls eine veränderte Menge an Polyenfettsäuren, insbesondere an oxygenierten Polyenfettsäuren im Saatöl synthetisiert wird, kann es sich im Prinzip um jede beliebige Pflanze handeln. Vorzugsweise ist es eine monokotyle oder dikotyle Nutzpflanze.

Beispiele für monokotyle Pflanzen sind die Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) gehören. Bei den dikotylen Nutzpflanzen sind u.a. zu nennen Leguminosen, wie Hülsenfrüchte und insbesondere Alfalfa, Sojabohne, Raps, Tomate, Zuckerrübe, Kartoffel, Zierpflanzen oder Bäume. Weitere Nutzpflanzen können beispielsweise Obst (insbesondere Äpfel, Birnen, Kirschen, Weintrauben, Citrus, Ananas und Bananen), Ölpalmen, Tee-, Kakao- und Kaffeesträucher, Tabak, Sisal, Baumwolle, Lein, Sonnenblume sowie Heilpflanzen und Weidegräser sowie Futterpflanzen sein. Besonders bevorzugt sind die Getreide Weizen, Roggen, Hafer, Gerste, Reis, Mais und Hirse, Futtergetreide, Zuckerrübe, Raps, Soja, Tomate, Kartoffel, Süßgräser, Futtergräser und Klee. Es ergibt sich von selbst, daß die Erfindung insbesondere übliche Nahrungs- bzw. Futterpflanzen betrifft. Hier sind neben den bereits erwähnten Pflanzen zusätzlich Erdnuß, Linse, Ackerbohne, Runkelrübe, Buchweizen, Möhre, Sonnenblume, Topinambur, Rübsen, Weißer Senf, Kohlrübe und Stoppelrübe zu nennen.

Besonders bevorzugt werden Ölsaaten.

Gegenstand der Erfindung sind ferner Vermehrungsmaterial und Ernteprodukte von erfindungsgemäßen Pflanzen, beispielsweise Samen, Früchte, Stecklinge, Knollen, Wurzelstöcke usw., sowie Teile dieser Pflanzen, wie Protoplasten, Pflanzenzellen und Kalli.

In einer weiteren Ausführungsform betrifft die Erfindung Wirtszellen, insbesondere prokaryontische und eukaryontische Zellen, die mit einem oben beschriebenen Nukleinsäuremolekül oder einem Vektor transformiert bzw. infiziert wurden, sowie Zellen, die von derartigen Wirtszellen abstammen und die beschriebenen Nukleinsäuremoleküle oder Vektoren enthalten. Die Wirtszellen können Bakterien, Viren, Algen, Hefe- und Pilzzellen sowie pflanzliche oder tierische Zellen sein.

Gegenstand der Erfindung sind auch solche Wirtszellen, die neben den erfindungsgemäßen Nukleinsäuremolekülen ein oder mehrere, auf gentechnologischem oder natürlichem Weg übertragene Nukleinsäuremoleküle enthalten, die die genetische Information für am LOX-abhängigen Katabolismus von mehrfach ungesättigten Fettsäuren in Pflanzen beteiligte Enzyme tragen.

Der vorliegenden Erfindung liegt außerdem die Aufgabe zugrunde, Verfahren zur Herstellung von Pflanzenzellen und Pflanzen, die sich durch einen veränderten Gehalt an Polyenfettsäuren, insbesondere an oxygenierten Polyenfettsäuren im Saatöl auszeichnen, bereitzustellen.

Diese Aufgabe wird durch Verfahren gelöst, mit deren Hilfe die Erzeugung neuer Pflanzenzellen und Pflanzen, die aufgrund der Übertragung der erfindungsgemäßen, für Acylhydrolase, insbesondere TAG-Lipase kodierenden Nukleinsäuremolekülen einen veränderten Gehalt an Polyenfettsäuren, insbesondere an oxygenierten Polyenfettsäuren im Saatöl aufweisen, möglich ist. Zur Erzeugung solcher neuer Pflanzenzellen und Pflanzen bieten sich verschiedene Methoden an. Zum einen können Pflanzen bzw. Pflanzenzellen mit Hilfe herkömmlicher gentechnologischer Transformationsmethoden derart verändert werden, daß die neuen Nukleinsäuremoleküle in das pflanzliche Genom integriert werden, d.h., es werden stabile Transformanten erzeugt. Zum anderen kann ein erfindungsgemäßes Nukleinsäuremolekül, dessen Anwesenheit und ggf. Expression in der Pflanzenzelle eine veränderte Biosyntheseleistung bewirkt, in der Pflanzenzelle bzw. der Pflanze als selbstreplizierendes System enthalten sein. So können die erfindungsgemäßen Nukleinsäuremoleküle beispielsweise in einem Virus enthalten sein, mit dem die Pflanze bzw. Pflanzenzelle in Kontakt kommt.

Erfindungsgemäß werden Pflanzenzellen und Pflanzen, die aufgrund der Expression einer erfindungsgemäßen Nukleinsäuresequenz einen veränderten Gehalt an Polyenfettsäuren, insbesondere an oxygenierten Polyenfettsäuren in Saatöl aufweisen, durch ein Verfahren hergestellt, das folgende Schritte umfaßt:
a) Herstellung eines rekombinanten Nukleinsäuremoleküls, umfassend die folgenden Bestandteilen in 5' -> 3'-Orientierung:
   - regulatorische Sequenzen eines in Pflanzenzellen aktiven Promotors,
   - operativ daran gebunden eine Nukleinsäuresequenz, die für ein Protein mit der biologischen Aktivität einer Acylhydrolase, insbesondere einer TAG-Lipase, besonders bevorzugt einer für oxygenierte Polyenfettsäuren spezifischen TAG-Lipase kodiert, und
   - ggf. operativ daran gebunden Sequenzen, die als Transkriptions-, Termination- und/oder Polyadenylierungssignale in Pflanzenzellen dienen können.
b) Übertragung des Nukleinsäuremoleküls aus a) auf pflanzliche Zellen.

Alternativ können eine oder mehrere erfindungsgemäße Nukleinsäuresequenzen als selbstreplizierendes System in die Pflanzenzelle bzw. die Pflanze eingebracht werden.

Als weitere Alternative kann Schritt a) des obigen Verfahrens dahingehend abgewandelt werden, daß die erfindungsgemäße Nukleinsäuresequenz, die für ein Protein mit der biologischen Aktivität einer Acylhydrolase, insbesondere einer TAG-Lipase, besonders bevorzugt einer für oxygenierte Polyenfettsäuren spezifischen TAG-Lipase kodiert, in Antisense-Orientierung an das 3'-Ende des Promotors gekoppelt ist.

Bei einem weiteren Aspekt der vorliegenden Erfindung können ein oder mehrere zusätzliche Nukleinsäuremoleküle eingeführt werden, die für Proteine kodieren, die die Übertragung von Fettsäuren auf Glyceride katalysieren (Transacylasen). Auf diese Weise können Lipide, deren oxygenierte Polyenfettsäuren durch die erfindungsgemäße TAG-Lipase abgepalten wurden, mit nicht oxygenierten polyungesättigten Fettsäuren substituiert werden, die beispielsweise für die Verwendung des resultierenden Pflanzenöls in der Lebensmittelindustrie geeignet sind.

Zur Vorbereitung der Einführung fremder Gene in höhere Pflanzen bzw. deren Zellen stehen eine große Anzahl von Klonierungsvektoren zur Verfügung, die ein Replikationssignal für E. *coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von E. *coli-Zellen* verwendet. Transformierte E. coli-Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden.

Voraussetzung für die Einführung der erfindungsgemäßen rekombinanten Nukleinsäuremoleküle und Vektoren in Pflanzenzellen ist die Verfügbarkeit geeigneter Transformationssysteme. Hier wurde während der letzten zwei Jahrzehnte ein breites Spektrum an Transformationsmethoden entwickelt und etabliert. Diese Techniken umfassen die Transformation pflanzlicher Zellen mit T-DNA unter Verwendung von *Agrobacterium tumefaciens* oder *Agrobacterium rhizogenes* als Transformationsmittel, Diffusion von Protoplasten, den direkten Gentransfer isolierter DNA in Protoplasten, die Injektion und Elektroporation von DNA in Pflanzenzellen, die Einbringung von DNA mittels der biolistischen Methoden sowie weitere Möglichkeiten, wobei der Fachmann die jeweils geeignete Methode problemlos ermitteln kann. Sämtliche Transformationsverfahren sind seit vielen Jahren gut etabliert und gehören zweifelsohne zum Standardrepertoire des Fachmanns in der pflanzlichen Molekularbiologie, Pflanzenbiotechnologie und Zell- und Gewebekultur.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode gewünschter Gene in die Pflanzenzelle können weitere DNA-Sequenzen erforderlich sein. Werden z.B. für die Transformation der Pflanzenzelle das Ti-oder Ri-Plasmid verwendet, so muss mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in E. *coli* als auch in Agrobakterien replizieren. Sie enthalten ein Selektionsmarkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt. Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker, Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls der eingesetzte Selektionsmarker nach erfolgter Transformation und Identifizierung erfolgreich transformierter Zellen bzw. Pflanzen wieder entfernt werden soll, stehen dem Fachmann hierfür verschiedene Strategien zur Verfügung. So können z.B. sequenzspezifische Rekombinasen verwendet werden, z.B. in Form der Retransformation einer Rekombinase-exprimierenden Ausgangslinie und Auskreuzung der Rekombinase nach erfolgter Entfernung des Selektionsmarkers (siehe z.B. Reiss *et al.* (1996) Proc. Natl. Acad. Sci. USA 93:3094-3098; Bayley *et al.* (1992) Plant Mol. Biol. 18:353-361; Lloyd *et al.* (1994) Mol. Gen. Genet. 242:653-657; Mäser *et al.* (1991) Mol. Gen. Genet. 230:170-176; Onouchi *et al.* (1991) Nucl. Acids Res. 19:6373-6378). Der Selektionsmarker kann auch durch Cotransformation mit anschließender Auskreuzung entfernt werden.

Die Regeneration der transgenen Pflanzen aus transgenen Pflanzenzellen erfolgt nach üblichen Regenerationsmethoden unter Verwendung üblicher Nährmedien und Phytohormone.

Die so erhaltenen Pflanzen können dann, falls erwünscht, mittels üblicher Verfahren, einschließlich molekularbiologischer Methoden, wie PCR, Blot-Analysen, oder biochemischer Verfahren auf Anwesenheit der eingeführten DNA, die ein Protein mit der enzymatischen Aktivität einer für oxygenierte Polyenfettsäuren spezifischen TAG-Lipase kodiert, bzw. auf Anwesenheit von TAG-Lipase-Enzymaktivität untersucht werden.

Unabhängig von den verwendeten regulatorischen Sequenzen, unter deren Kontrolle die Expression der erfindungsgemäßen Acylhydrolase-, insbesondere TAG-Lipase-DNA-Sequenzen steht, steht dem Fachmann ein breites Spektrum an molekularbiologischen und/oder biochemischen Verfahren für die Analyse der transformierten Pflanzenzellen, transgenen Pflanzen, Pflanzenteile, Ernteprodukte und Vermehrungsmaterial zur Verfügung, z.B. PCR, Northern Blot-Analyse zum Nachweis von für die erfindungsgemäße TAG-Lipase spezifischer RNA bzw. zur Bestimmung der Höhe der Akkumulation von TAG-Lipasespezifischer RNA, Southern Blot-Analyse zur Identifizierung von erfindungsgemäßen TAG-Lipase-kodierenden DNA-Sequenzen oder Western Blot-Analyse zum Nachweis des durch die erfindungsgemäßen Nukleinsäuremoleküle kodierten TAG-Lipase. Selbstverständlich kann der Nachweis der enzymatischen Aktivität der erfindungsgemäßen TAG-Lipase auch vom Fachmann mittels in der Literatur erhältlicher Protokolle bestimmt werden. Weiter kann man z.B. den durch Selbstung oder Kreuzungen erhaltenen Samen auf Medium auslegen, das das zu dem zusammen mit der TAG-Lipase-DNA-Sequenz übertragenen Selektionsmarker passende Selektionsmittel enthält, und anhand der Keimfähigkeit und des Wachstums der Tochtergeneration(en) und des Segregationsmusters Rückschlüsse auf den Genotyp der jeweiligen Pflanze schließen.

Eine weitere Aufgabe der Erfindung besteht darin, Verwendungen der erfindungsgemäßen Nukleinsäuresequenzen sowie der sie enthaltenden Nukleinsäuremoleküle aufzuzeigen.

Diese Aufgabe wird durch die erfindungsgemäßen Verwendungen der neuen DNA-Sequenzen und -Moleküle zur Erzeugung von Pflanzenzellen und Pflanzen, die sich durch einen im Vergleich zu Wildtypzellen bzw. Wildtyppflanzen veränderten Gehalt insbesondere an Polyenfettsäuren, besonders bevorzugt an oxygenierten Polyenfettsäuren im Saatöl auszeichnen, gelöst.

Die vorliegende Erfindung umfaßt ferner jede mögliche Form des Einsatzes der erfindungsgemäßen Nukleinsäuremoleküle, deren Gegenwart und ggf. Expression in Pflanzen eine Veränderung des Gehalts an Polyenfettsäuren, insbesondere an oxygenierten Polyenfettsäuren im Saatöl bewirkt, sowie des Einsatzes der erfindungsgemäßen Proteine oder Fragmente davon, deren enzymatische Aktivität eine solche Veränderung herbeiführt.

Weiter betrifft die Erfindung die Verwendung einer erfindungsgemäßen DNA-Sequenz oder Teile davon zur Identifizierung und Isolierung homologer Sequenzen aus Pflanzen oder anderen Organismen.

Die erfindungsgemäßen Nukleinsäuremoleküle können somit erfindungsgemäß verwendet werden, um transgene Pflanzen zu erzeugen, in denen der Gehalt an den erfindungsgemäßen Acylhydrolasen, insbesondere TAG-Lipasen höher oder geringer ist als natürlicherweise, oder in Zelltypen oder Entwicklungsstufen vorliegt, bei denen die erfindungsgemäßen Acylhydrolasen, insbesondere TAG-Lipasen normalerweise nicht gefunden werden. Dies bewirkt eine Änderung des Gehalts an Triacylglycerol und Cholesterylestern in diesen Zellen.

Die Akkumulierung von Fettsäuren mit ungewöhnlichen Strukturen kann ein vorteilhafter Phenotyp in Pflanzen sein, die für Lebensmittel verwendet werden. Triacylglycerol-Lipasen können ebenfalls nützlich bei der Verarbeitung von Pflanzenölen und der Entwicklung von neuen Saatölen sein, da durch eine selektive Abspaltung von oxidierten Poleinfettsäureresten durch die erfindungsgemäßen TAG-Lipasen die Haltbarkeit von Pflanzensaatölen für die Lebensmittelindustrie verlängert werden kann.

Für manche Anwendungen kann es nützlich sein, die erfindungsgemäßen TAG-Lipasen in unterschiedliche zelluläre Kompartimente einzuführen oder deren Sekretion aus der Zelle zu erleichtern. Es ist somit möglich, daß die erfindungsgemäßen Nukleinsäuremoleküle derart modifiziert sind, daß die erfindungsgemäßen Nukleinsäuremoleküle mit geeigneten intrazellulären Target-Sequenzen, wie Transit-Sequenzen (K. Keegstra (1989) Cell 56: 247-253), Signal-Sequenzen und dergleichen ergänzt werden.

Für manche Anwendungen kann es auch erwünscht sein, die Expression von Nukleinsäuremolekülen zu vermindern oder zu eliminieren, die für TAG-Lipasen, insbesondere für TAG-Lipasen, die für oxygenierte Polyenfettsäuren spezifisch sind, kodieren. Um dies zu erreichen, kann ein für die Cosupression der erfindungsgemäßen TAG-Lipase entwickeltes Nukleinsäuremolekül durch Verknüpfen eines Gens oder Genfragments, das eine für oxygenierte Polyenfettsäuren spezifische TAG-Lipase kodiert, mit Pflanzenpromotersequenzen, erzeugt werden. Alternativ kann ein Nukleinsäuremolekül, das für die Expression von Antisense-RNA für das gesamte oder einen Teil des erfindungsgemäßen Nukleinsäuremoleküls entwickelt ist, durch Verbinden des Gens oder Genfragments in reverser Orientierung zu Pflanzenpromotersequenzen erzeugt werden. Sowohl die Gene für die Cosuppression als auch die Antisense-Gene können mittels Transformation in die Pflanzen eingeführt werden, wodurch die Expression der entsprechenden endogenen Gene vermindert oder eliminiert ist.

Die Erfindung basiert auf der erfolgreichen Isolierung von für eine Acylhydrolase, insbesondere für eine TAG-Lipase, besonders bevorzugt für eine für oxygenierte Polyenfettsäuren spezifischen TAG-Lipase kodierenden cDNA-Klonen aus einer cDNA-Bibliothek aus *Arabidopsis thaliana.* Die Sequenzen dieser cDNA-Klone, die ein vollständiges Leseraster umfassen, sind in SEQ ID NO: 1, SEQ ID No:3 und SEQ ID No:5 dargestellt. Unter Verwendung dieser Sequenzen gelang erstmals die Erzeugung transgener Pflanzen, die aufgrund der Übertragung und Expression einer TAG-Lipase, insbesondere einer für oxygenierte Polyenfettsäuren spezifischen TAG-Lipase einen gegenüber Wildtyppflanzen veränderten Gehalt an oxygenierten Polyenfettsäuren im Saatöl aufweisen.

Die nachfolgenden Beispiele dienen zur Erläuterung der Erfindung, ohne diese in irgendeiner Weise einzuschränken.

### Beispiele

### Beispiel 1:

### Klonierung und Expression einer für die TAG-Lipase kodierenden cDNA aus Arabidopsis thaliana

cDNA-Sequenzen, die für eine TAG-Lipase aus *Arabidopsis thaliana* kodieren, wurde mit Hilfe einer Durchmusterung von wie im folgenden beschrieben hergestellten cDNA-Bibliotheken aus *Arabidopsis thaliana* gemäß eines ebenfalls im folgenden beschriebenen standardisierten Protokolls identifiziert. Drei der annotierten Sequenzen (Lipase 1: SEQ ID No:1, Lipase 2: SEQ ID No:3, Lipase 3: SEQ ID No:5) wurden kloniert, exprimiert und auf Aktivität getestet.

### I. Herstellung von verschiedenen cDNA-Banken aus Arabidopsis thaliana

Nach dem im folgenden beschriebenen Protokoll wurde cDNA-Banken aus einem Gemisch verschiedener Organe von *Arabidopsis* sowie eine cDNA-Bank aus Blüten von *Arabidopsis* hergestellt.

### a) Isolation von RNA aus Arabidopsis

Zur Isolation von RNA aus *Arabidopsis* wurden die folgenden Reagenzien verwendet:
- Extraktionspuffer I:
   100 mM Tris/HCl pH 7,5
   25 mM EDTA
   2 % Laurylsarkosyl
   4 M Guanidiniumthiocyanat
   5 % (w/v) PVP (Polyklar) (wird als unlösliche Substanz erst bei Versuchsanfang in die fertige Lösung gegeben)
   1 ml/100 ml β-Mercaptoethanol (wird kurz vor der Benutzung des Extraktionspuffers zugegeben)
- PCI:
   20 ml Phenol
   20 ml Chloroform
   1 ml Isoamylalkohol (mit H₂O gesättigt)
- Chloroform
- 70 % Ethanol, 100 % Ethanol
- DEPC-behandeltes H₂O
- Extraktionspuffer II (mit DEPC behandeltem Wasser angesetzt):
   100 ml Tris/HCl pH 8,8
   100 mM NaCl
   5 mM EDTA
   2 % SDS

Die Durchführung der Isolation von RNA aus *Arabidopsis* wird nach dem folgenden Protokoll durchgeführt:
- Zermörsern von 20 g Pflanzenmaterial in flüssigem Stickstoff
- Vorbereitung von 100 ml Extraktionspuffer I mit PVP und Mercaptoethanol, Zugabe des zermörserten Pflanzenmaterials und sofortige Mischung.
- Nach der Homogenisierung Überführung der Lösung in Falcon-Tubes und Schütteln für ca. 15 Minuten
- Abzentrifugieren für 10 bis 15 Minuten bei 4.500 bis 5.500 rpm in Falcon-Tubes in einer Sigmazentrifuge
- Kippen des Überstandes in frische Falcons und Verwerfen des Pellets
- Versetzen mit 1 vol. PCI und Schütteln für 15 Minuten, 15 Minuten Stehenlassen auf Eis und abzentrifugieren, Abnehmen des Überstandes und Überführung in neue Falcons
- Abnehmen des Überstandes und Versetzten mit 1 vol. Chloroform, Schütteln für 15 Minuten und abzentrifugieren
- Abnehmen des Überstandes und Versetzen mit 1 vol. Isopropanol, Fällen über Nacht bei -20°C
- Abzentrifugieren für 30 Minuten bei 9.000 rpm
- Lösen der Pellets in je 20 ml Extraktionspuffer II und Versetzen mit 1 vol. Isopropanol
- Inkubieren für eine Stunde bei -20°C
- Abzentrifugieren für 30 Minuten bei 9.000 rpm
- Zweimaliges Waschen der Pellets mit 1 ml 70 % Ethanol
- Trocknen der Pellets in einer Speed-Vac
- Aufnehmen der Pellets in 1.500 ml H₂O, Stehenlassen auf Eis zum Lösen für 30 Minuten, Abnehmen des Überstandes und Überführung in frische Eppendorf-Caps, kalt stehenlassen
- Vermessen der RNA im Photometer bei einer Verdünnung von 1:100,
   Bei der Messung sollte das Verhältnis von A₂₆₀ zu A₂₈₀ größer/gleich 1,8 sein. Der Meßwert wird mit dem Verdünnungsfaktor und einem Faktor 40 multipliziert, wodurch sich die Konzentration der RNA in µg/ml ergibt. Die Durchführung eines Wavelength-Scans von 200 - 300 nm ergibt eine typische RNA-Kurve mit einem Maximum bei 260 nm. Anschließend wird die Absolutmenge an RNA berechnet und zur Ermittlung der Qualität der RNA ein RNA-Agarosegel durchgeführt.

### b) mRNA-Isolation

Die mRNA-Isolierung wird mit dem mRNA-Isolierungskit Poly-Attract von Promega (Mannheim, Deutschland) durchgeführt.

### c) RT-PCR

Die reverse Transkriptase-PCR wurde mit 1 µg RNA, 200 pmol oligo-dT mit Superscript II von Gibco (Eggenstein, Deutschland) gemäß der Vorschrift des Herstellers durchgeführt.

### II. PCR-Amplifikation

Die PCR-Amplifikation wurde mit dem Expand^{TM} High Fidelity PCR-System gemäß der Vorschrift des Herstellers (Boehringer Mannheim, Deutschland) durchgeführt. Als Template wurden für die Lipasen 1 und 2 das *Arabidopsis* cDNA-Gemisch und für die Lipase 3 die cDNA aus der Blüte von *Arabidopsis* verwendet. Als Primer wurden die folgenden Sequenzen verwendet:
Lipase 1: (3 x Methionin im Anfangsbereich der Sequenz) 5':-CCC GCATGC ATG CAG TTG TCT CCG GAA CGA TGC (A1) 5':-GGG GCATGC ATG TCT GAA AAC AGA GAG GCT TGG (A2) 5':-AAA GCATGC ATG GAG CTG CTT CAC GGC TCC AAC (A3) 3':-AAA GTCGAC TCA AAA AGG GCT GAC CCT GCC AGC C
Lipase 2: 5':-CCC CGC ATG CAT GGC TTC TTC ACT GAA GAA 3':-AAA CTG CAG TTA TGT ATC CAC TGT ACC AGA GCC AAG
Lipase 3:

5':-GGG GCATGC ATG TGT AGA AGA TAT TTC GTG CAT AGT

3':-TTT CTGCAG TGA CAG ATG AGG TTT GCC TGT TTC C

Für die Herstellung von Expressionsklonen, die die rekombinante TAG-Lipase in E. *coli* überexprimieren, wurde das offene Leseraster für das vollständige unprozessierte Protein mittels der vorstehenden Oligo-Nukleotidprimer von den DNA-Sequenzen gemäß den Sequenzen SEQ ID No:1, 3 und 5 mittels PCR amplifiziert. Die PCR-Bedingungen waren wie folgt: 2 Minuten Denaturierung bei 94°C, dann für 10 Zyklen: 30 Sekunden Denaturierung bei 94°C, 30 Sekunden Annealing bei 65°C, 3 Minuten Elongation bei 72°C; dann für 15 Zyklen: 30 Sekunden Denaturierung bei 94°C, 30 Sekunden Annealing bei 65°C, 3 Minuten Elongation bei 72°C plus einem Zeitinkrement von 5 Sekunden pro Zyklus; zum Schluß weitere 2 Minuten Elongation bei 72°C.

Das amplifizierte PCR-Fragment wurde in den Expressionsvektor QIAexpress pQE 30 hineinligiert und mit Transformation in XL1-Blue-Zellen unter Nutzung des p GEM^{R}-T Easy Vector System II Kits (Promega, Madison, USA) vorkloniert. Anschließend erfolgte die Umklonierung in den Expressionsstamm E.coli SG13009[pREP4].

### IV. Für die Expression der pflanzlichen TAG-Lipase wurde der E.coli-Stamm in LB-Medium bei 10°C für eine Dauer von 72 Stunden angezogen.

### V. Reinigung für membranassoziierte Proteine

Die Reinigung erfolgte nach den folgenden Schritten:
- Herstellung einer Membranfraktion der E.coli SG13009[pREP4]
- Solubilisierung in Natriumphosphat bei pH 8,0, 1 M NaCl, 0,5 % Triton - 1 h Zentrifugieren bei 4°C und 37.000 rpm
- Reinigung des Überstands mittels eines Talon metal affinity resins (Clontech, Palo Alto, USA) gemäß den Instruktionen des Herstellers
- Eluierung des Proteins mit Natriumphosphat, 1 M NaCl bei pH 5,0

### Beispiel 2: Bestimmung der TAG-Lipase-Aktivität

Zur Bestimmung der TAG-Lipase-Aktivität wurde das wie vorstehend beschrieben erhaltene Proteineluat mit einem Lipidextrakt (Chloroform:Methanol, Bligh/Dyer 1959) aus vier Tage alten Keimlingen von *Cucumis sativa* 40 Minuten bei 25°C unter Zugabe von Pufferkonzentrat (1 M Tris pH 8,5; 0,5 M NaCl; 50 mM CaCl₂) zur Einstellung des pH-Wertes auf 8,0 inkubiert. Anschließend wurde mit Essigsäure angesäuert und mit Hexan extrahiert. Die Analyse erfolgte mittels HPLC (LiCHrosphere 100 RP-18 (MERCK, Darmstadt, Deutschland); Laufmittel: Acetonitril:H₂O:Essigsäure 70:30:0,1).

### Beispiel 3: Transformation von Arabidopsis thaliana- und Nicotiana tabacum-Pflanzen und Regeneration intakter Pflanzen

Für die Herstellung transgener Pflanzen, die die TAG-Lipase-Sequenz überexprimieren und daher eine gegenüber nicht-transformierten Pflanzen erhöhte Aktivität des Enzyms TAG-Lipase aufweisen, wurden die DNA-Sequenzen gemäß SIQ ID No:1, 3 und 5 jeweils in einen geeigneten binären Vektor (z.B. pPCV001, pPCV002 (Koncz & Schell 1986 MGG 204:383-396) kloniert.

Anstelle des genannten Vektors kann jeder beliebige, für die Pflanzentransformation geeigneter Vektor für die Herstellung eines chimären Gens, bestehend aus einer Fusion des CaMV 35S-Promotors oder eines anderen konstitutiven, samenspezifischen, keimungsspezifischen oder blütenspezifischen Promotors, der die Transkription und, falls erwünscht, Translation in Pflanzenzellen gewährleistet, und DNA-Sequenzen, die für TAG-Lipase kodieren, verwendet werden.

Der binäre Vektor wurde dann in *Agrobacterium tumefaciens* (Stamm GV2260; Horsch et al. (1985), Science 227, 1229-1231) transformiert und zur Transformation von Arabidopsis- bzw. Tabakpflanzen (SNN) mittels der Blattscheiben-Transformationstechnik (Horsch et al., supra) eingesetzt.

Hierzu wurde eine Übernachtkultur des entsprechenden *Agrobacterium tumefaciens-*Klons für 10 Minuten bei 5000 rpm abzentrifugiert, und die Bakterien wurden in 2YT-Medium resuspendiert. Blattstücke von Sterilkulturen von *Arabidopsis thaliana* bzw. *Nicotiana tabacum* cv. *Samsun* NN) wurden kurzzeitig in die Bakteriensuspension gelegt. Die Blattstücke wurden anschließend auf MS-Medium (Murashige und Skoog (1962), Physiol. Plant. 15, 473; 0,7% Agar) gelegt und zwei Tage im Dunkeln inkubiert. Anschließend wurden die Blattstücke zur Sproßinduktion auf MS-Medium (0,7% Agar) mit 1,6% Glukose, 1 mg/l 6-Benzylaminopurin, 0,2 mg/l Naphthylessigsäure, 500 mg/l Claforan (Cefotaxim, Hoechst, Frankfurt) und 50 mg/l Kanamycin gelegt. Das Medium wurde alle sieben bis zehn Tage gewechselt. Wenn sich Sprosse entwickelt hatten, wurden die Blattstücke in Glasgefäße, die das selbe Medium enthielten, überführt. Entstehende Sprosse wurden abgeschnitten und auf MS-Medium mit 2% Saccharose und 250 mg/l Claforan gegeben und zu ganzen Pflanzen regeneriert.

Die Analyse der erhaltenen transgenen Arabidopsis- und Tabakpflanzen bestätigte die erfolgreiche Veränderung des Lipidgehalts durch Expression der erfindungsgemäßen Nukleinsäuremoleküle.

## Patentansprüche

1. Rekombinantes Nukleinsäuremolekül, umfassend
a) regulatorische Sequenzen eines in Pflanzenzellen aktiven Promotors;
b) operativ daran gebunden eine DNA-Sequenz, die ein Protein mit der enzymatischen Aktivität einer Acylhydrolase kodiert; und
c) operativ daran gebunden regulatorische Sequenzen, die als Transkriptions-, Terminations- und/oder Polyadenylierungssignale in Pflanzenzellen dienen können.

2. Rekombinantes Nukleinsäuremolekül nach Anspruch 1, wobei die DNA-Sequenz ein Protein mit der enzymatischen Aktivität einer Triacylglycerin-Lipase (TAG-Lipase) kodiert.

3. Rekombinantes Nukleinsäuremolekül nach Anspruch 2, wobei die DNA-Sequenz ein Protein mit der enzymatischen Aktivität einer für oxygenierte Polyenfettsäuren spezifischen TAG-Lipase kodiert

4. Rekombinantes Nukleinsäuremolekül nach einem der Ansprüche 1 bis 3, **dadurch gekennzeichnet, daß** die DNA-Sequenz für eine Acylhydrolase, insbesondere eine TAG-Lipase, aus *Arabidopsis thaliana* kodiert.

5. Rekombinantes Nukleinsäuremolekül nach einem der Ansprüche 1 bis 4, enthaltend eine DNA-Sequenz, die ausgewählt ist aus der Gruppe, bestehend aus
a) DNA-Sequenzen, die die in SEQ ID No. 3 angegebene Nukleotidsequenz umfassen,
b) DNA-Sequenzen, die die in SEQ ID No. 4 angegebene Aminosäuresequenz oder Fragmente davon kodieren,
c) DNA-Sequenzen, die eine Nukleotidsequenz, die mit einem komplementären Strang der Nukleotidsequenz von a) oder b) hybridisieren, oder Fragmente dieser Nukleotidsequenz umfassen,
d) DNA-Sequenzen, die eine Nukleotidsequenz, die zu einer Nukleotidsequenz von c) degeneriert ist, oder Fragmente dieser Nukleotidsequenz umfassen,
e) DNA-Sequenzen, die ein Derivat, Analog oder Fragment einer Nukleotidsequenz von a), b) oder c) darstellen.

6. Rekombinantes Nukleinsäuremolekül nach einem der Ansprüche 1 bis 5, wobei der Promotor ein konstitutiver Promotor, vorzugsweise der 35S RNA-Promotor von CaMV ist.

7. Vektor, umfassend ein rekombinantes Nukleinsäuremolekül nach einem der vorangehenden Ansprüche.

8. Wirtszelle, enthaltend ein rekombinantes Nukleinsäuremolekül oder einen Vektor nach einem der vorangehenden Ansprüche.

9. Rekombinantes Protein mit der enzymatischen Aktivität einer Acylhydrolase.

10. Rekombinantes Protein nach Anspruch 9, wobei die Acyhydrolase eine TAG-Lipase ist.

11. Rekombinantes Protein nach Anspruch 9 oder 10, wobei die Acyhydrolase eine für oxygenierte Polyenfettsäuren spezifische TAG-Lipase ist.

12. Rekombinantes Protein nach einem der Ansprüche 9 bis 11 mit der enzymatischen Aktivität einer in *Arabidopsis* hergestellten Acylhydrolase.

13. Protein nach einem der Ansprüche 9 bis 12, enthaltend eine Aminosäuresequenz, ausgewählt aus Aminosäure-Sequenzen, die die in SEQ ID No. 4 angegebene Aminosäuresequenz oder Fragmente davon umfassen.

14. Verfahren zur Herstellung von Pflanzen bzw. Pflanzenzellen, umfassend die folgenden Schritte:
a) Herstellung einer Nukleinsäuresequenz, bestehend aus den folgenden Bestandteilen, die in der 5'-3'-Orientierung aneinandergereiht sind:
- ein in Pflanzen funktionsfähiger, insbesondere samenspezifischer, Promotor,
- mindestens eine DNA-Sequenz, die ein Protein mit der enzymatischen Aktivität einer Acylhydrolase, insbesondere einer TAG-Lipase, besonders bevorzugt einer für oxygenierte Polyenfettsäuren spezifischen Triacylglycerin-TAG-Lipase kodiert, und
- gegebenenfalls ein Terminationssignal für die Termination der Transkription und die Addition eines poly-A-Schwanzes an das entsprechende Transkript, sowie ggf. davon abgeleitete DNA-Sequenzen;
b) Übertragung der Nukleinsäuresequenz aus a) auf pflanzliche Zellen und gegebenenfalls Integration der Nukleinsäuresequenz in das pflanzliche Genom.

15. Transgene Pflanzenzelle, enthaltend ein rekombinantes Nukleinsäuremolekül oder einen Vektor nach einem der Ansprüche 1 bis 7 oder hergestellt nach einem Verfahren nach Anspruch 14.

16. Pflanzenzelle nach Anspruch 15, die mindestens ein weiteres Fremdgen enthält.

17. Pflanzenzelle nach Anspruch 15 oder 16, einschließlich Protoplasten, die einen gegenüber nicht-transformierten Pflanzenzellen veränderten, insbesondere erhöhten, Acylhydrolase, insbesondere TAG-Lipase-Gehalt aufweist.

18. Transgene Pflanzen, enthaltend eine Pflanzenzelle nach einem der Ansprüche 15 bis 17 oder hergestellt nach Anspruch 14, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

19. Pflanzen nach Anspruch 18, die einen gegenüber Wildtyppflanzen veränderten Acylhydrolase-, insbesondere TAG-Lipase-Gehalt aufweisen.

20. Pflanzen nach Anspruch 19, die einen gegenüber Wildtyppflanzen erhöhten TAG-Lipase-Gehalt aufweisen.

21. Pflanzen nach einem der Ansprüche 18 bis 20, die einen gegenüber Wildtyppflanzen veränderten Gehalt an Polyenfettsäuren, insbesondere an oxygenierten Polyenfettsäuren im Samenöl enthalten.

22. Pflanzen nach Anspruch 21, die einen gegenüber Wildtyppflanzen verminderten Gehalt an Polyenfettsäuren, insbesondere an oxygenierten Polyenfettsäuren im Samenöl enthalten.

23. Dikotyle Pflanzen nach einem der Ansprüche 18 bis 22.

24. Pflanzen nach Anspruch 23, bei denen es sich um Nutzpflanzen, Nahrungs- und/oder Futterpflanzen handelt.

25. Monokotyle Pflanzen nach einem der Ansprüche 13 bis 17.

26. Pflanzen nach Anspruch 25, bei denen es sich um Nutzpflanzen, Nahrungs- und/oder Futterpflanzen handelt.

27. Verwendung einer Pflanze nach einem der Ansprüche 18 bis 26 als Nutz-, Nahrungs- oder Futterpflanze.

28. Verwendung einer Nukleinsäuresequenz oder eines Vektors nach einem der Ansprüche 1 bis 7 zur Herstellung transgener Pflanzen bzw. Pflanzenzellen.

29. Verwendung nach Anspruch 28, wobei die Pflanzen bzw. Pflanzenzellen einen veränderten Acylhydrolase-, insbesondere TAG-Lipase-Gehalt aufweisen.

30. Verwendung nach Anspruch 28 oder 29, wobei die Pflanzen bzw. Pflanzenzellen einen veränderten Gehalt an Polyenfettsäuren, insbesondere an oxygenierten Polyenfettsäuren im Samenöl enthalten.

31. Verfahren zur Auffindung von Nukleinsäuresequenzen, die ein Protein mit der enzymatischen Aktivität einer Acylhydrolase, insbesondere einer TAG-Lipase, besonders bevorzugt einer für oxygenierte Polyenfettsäuren spezifischen TAG-Lipase kodiert, umfassend
a) Testen einer cDNA- oder genomischen Bank mit der Nukleinsäuresequenz nach einem der Ansprüche 1 bis 6,
b) Identifizierung eines DNA-Klons, der mit der Nukleinsäuresequenz nach einem der Ansprüche 1 bis 6 hybridisiert,
c) Isolierung des in Schritt b) identifizierten DNA-Klons, und
d) Sequenzierung des cDNA- oder genomischen Fragments, das den in Schritt c) isolierten Klon umfaßt.

32. Verfahren zur Veränderung des Gehalts an Polyenfettsäureresten, insbesondere an oxidierten Polyenfettsäureresten in transgenen Pflanzen durch Beeinflussung des Lipoxygenase (LOX)-abhängigen Stoffwechsels von mehrfach ungesättigten Fettsäuren in transgenen Pflanzen.

33. Verfahren nach Anspruch 32, wobei die Veränderung des LOX-abhängigen Stoffwechsels von mehrfach ungesättigten Fettsäuren durch Modifizierung der Expression von TAG-Lipase erfolgt.

34. Verfahren nach Anspruch 33, umfassend
a) gegebenenfalls Transformieren einer Wirtszelle mit LOX;
b) Transformieren einer Wirtszelle mit dem Nukleinsäuremolekül nach einem der Ansprüche 1 bis 6; und
c) Wachstum der in Schritt a) und b) hergestellten transformierten Wirtszelle unter Bedingungen, die für die Expression des Nukleinsäuremoleküls nach einem der Ansprüche 1 bis 6 geeignet sind.

35. Verwendung einer TAG-Lipase, kodiert durch ein Nukleinsäuremolekül nach einem der Ansprüche 1 bis 6, zur Verarbeitung von pflanzlichen Ölen, die oxidierte Polyenfettsäurereste umfassen.

36. Verwendung nach Anspruch 35 zur spezifischen Isolierung der oxidierten Polyenfettsäurereste aus den pflanzlichen Ölen.

37. Verwendung nach Anspruch 35 oder 36 zur Verminderung des Gehalts an oxidierten Polyenfettsäureresten in Pflanzenöl.
